# EUROPÄISCHE PATENTSCHRIFT

(11) **EP 0 949 909 B1**
(45) Veröffentlichungstag und Bekanntmachung des Hinweises auf die Patenterteilung: **09.01.2002**
(21) Anmeldenummer: 97952796.7
(22) Anmeldetag: 21.11.1997
(51) Int. Cl.: A61K 9/20, A61K 9/00, A61K 9/70

(54) **EINZELN DOSIERTE, BEI KONTAKT MIT FLÜSSIGKEIT SCHNELL ZERFALLENDE, WIRKSTOFF- UND INSBESONDERE AROMASTOFFHALTIGE, FOLIENFÖRMIGE DARREICHUNGSFORM**
INDIVIDUALLY DOSED FOIL-FORM PRESENTATION WHICH DECOMPOSES RAPIDLY ON CONTACT WITH LIQUID AND CONTAINS AN ACTIVE SUBSTANCE, IN PARTICULAR AN AROMATIC SUBSTANCE
FORME GALENIQUE A DOSAGE INDIVIDUEL, SOUS FORME PELLICULAIRE, CONTENANT UN PRINCIPE ACTIF ET NOTAMMENT UNE SUBSTANCE AROMATIQUE, SE DESAGREGEANT RAPIDEMENT AU CONTACT DE LIQUIDE

(30) Priorität: 16.12.1996 DE 19652257
(43) Veröffentlichungstag der Anmeldung: 20.10.1999
(73) Patentinhaber: LTS LOHMANN Therapie-Systeme AG, 56626 Andernach (DE)
(72) Erfinder: HORSTMANN, Michael, D-56564 Neuwied (DE); LAUX, Wolfgang, D-65582 Diez (DE)
(74) Vertreter: Flaccus, Rolf-Dieter, Dr.
(86) Internationale Anmeldenummer: EP9706533
(87) Internationale Veröffentlichungsnummer: WO9826764

(56) Entgegenhaltungen:
- EP-A- 0 303 445
- EP-A- 0 460 588
- DATABASE WPI Section Ch, Week 9445 Derwent Publications Ltd., London, GB; Class A96, AN 94-221789 XP002062879 & JP 06 157 327 A (SEKISUI) , 3.Juni 1994

## Beschreibung

Die vorliegende Erfindung betrifft eine einzeln dosierte, bei Kontakt mit Flüssigkeit schnell zerfallende, wirkstoff- und insbesondere aromastoffhalige, folienförmige Darreichungsform, bei der sich der Aromastoff als innere, fettlösliche Phase in Form von flüssigen Tröpfchen in einer äußeren, festen wasserlöslichen Phase verteilt befindet.

Im Mundbereich und auf den Schleimhäuten des Mundes anzuwendende flächige Darreichungsformen sind bekannt.
US 3,444,858 beschreibt Medikamentstreifen auf Basis eines gelatineartigen Materials. Auch sind zu Anfang der 70er Jahre bereits Arzneimittel in Folienform beschrieben worden, wie z.B. im New England Journal of Medicine, 289, 533-535 (1973). Die DE 24 49 865 beschreibt Arzneimittelwirkstoffträger in Folienform, die unterschiedliche Wirkstoffe und Wirkstoffkonzentrationen enthalten.
Die US 4,128,445 offenbart technische Lösungen bei der Beladung von Trägermaterial mit Wirkstoffen und geht dabei auf die nachträgliche Zugabe von Wirkstoffzubereitungen auf vorgefertigte folienförmige Zubereitungen ein. Es werden Beladungsverfahren in trockener und feuchter Form beschrieben, die eine gleichmäßige, nachträgliche Verteilung von Wirkstoff auf einer Schicht zum Ziel haben. In der kanadischen Patentanmeldung Nr. 492 040 wird ein Prozeß zur Herstellung von folienförmigen Zubereitungen unter Einsatz von Wirkstoff mit Gelatine oder Agar, Gluten, Carboxyvinylpolymer, mehrwertigem Alkohol, pflanzlichem Schleim, Wachs oder Wasser beschrieben.

Bekannt sind auch Anwendungsvorschläge für wirkstoffbeladene Folien außerhalb des Arzneimittelbereiches. So wird in der EP 0 219 762 eine wasserlösliche Folie aus Stärke, Gelatine, Glyzerin oder Sorbit offenbart, die mittels Walzenauftragsverfahren beschichtet ist. Dabei wird erwähnt, daß sich solche Dosierungsformen auch mit Inhaltsstoffen von chemischen Reagenzien, Aromastoffen und dergleichen herstellen lassen.

Die DE 36 30 603 sieht vor, eine flächige Dosierungsform auf einem Trägermaterial (Trennfilm ) dosisweise abziehbar zu gestalten.

Arzneimittelhaltige folienförmige Systeme und deren Vorteile sind weiterhin aus der US 5,047,244 mit einem zweischichtigen Aufbau aus einer wasserquellbaren Schicht und einem wasserunlöslichen Barrierefilm bekannt. Die Verwendung von Polymeren wie Polyethylenglycol, der Einsatz von kolloidalem Siliciumdioxid, von bioadhäsiven (z.B. carboxyfunktionellen ) Polymeren, aber auch von Polyvinylalkohol und einer Reihe anderer Hilfsstoffe ist daraus bereits ebenfalls bekannt.

Eine zur Herstellung folienförmiger aromastoffhaltiger Zubereitungen geeignete Formulierung wird in der EP 0 460 588 beschrieben. Besondere Vorteile werden in der Zusammensetzung aus 20 bis 60 Gew.% Filmbildner, 2 bis 40 Gew.% Gelbildner, 0,1 bis 35 Gew.% Wirkstoff- bzw. Aromastoff und maximal 40 Gew.% eines inerten Füllstoffes gesehen. Als Gelbildner ist Polyvinylalkohol neben anderen Einsatzstoffen erwähnt. Es erweist sich jedoch, daß die gelbildenden Eigenschaften von Polyvinylalkohol mit den in dieser Schrift genannten Filmbildnern nur bedingt verträglich sind. Ein Anteil von 20 und mehr Gewichtsanteilen Filmbildner - zumeist ein Zuckerderivat, Polyethylenglycol etc. - führte zu erheblichem Aromaverlust bereits bei der herstellungsbedingten Trocknung in dünner Schicht.

Mikrokapseln sind bekannte Anwendungsformen zum Schutz von flüchtigen oder inkompatiblen, feinzerteilten Produkten durch Umhüllung mit fester Phase (z.B. Bauer/Frömming, Pharmazeutische Technologie, Stuttgart 1986, 563-566). Bei mikroverkapselten Aromastoffen werden einzelne Flüssigkeitstropfen durch Umhüllung verarbeitbar, z.B. rieselfähig gemacht. Solche Formen wurden bereits für die Anwendung im Mundbereich vorgeschlagen z.B. gemäß US 5,286,496. Es handelt sich dabei jedoch um feinkörnige Zwischenprodukte zur Fertigung von größer dimensionierten Endprodukten.

Aromastoffhaltige flächenförmige Darreichungsformen zur Anwendung im Mundbereich sind auch aus der EP 0 452 446 bekannt, jedoch werden keine Maßnahmen beschrieben, wie eine Aromastoffverdampfung bei der Herstellung und/oder Lagerung verhindert werden kann.

Zwar nicht bei folienförmiger, aber bei als flache, feste offenporige Schäume erscheinenden Formen wurde entsprechend der US 4,946,684 die Verwendung von Zuckeralkoholen zur Erhöhung der Feuchtestabilität vorgeschlagen. Nach eigenen Erkenntnissen führen jedoch bei der Verarbeitung von Aromastoffen hohe Anteile solcher die Löslichkeit steigernder Zusatzstoffe zu größerem Aromaverlust.

Den bekannten Verfahren zur Herstellung und zum Aufbau folienförmiger Träger mit Aromastoff haften demnach grundsätzliche Nachteile an:
Zum einen ist die mechanische Festigkeit unbefriedi-gend; insbesondere ist die Biegefestigkeit und die Reißfestigkeit der erhaltenen Folien nicht hinreichend für verbraucherfreundliche Routineanwendungen.
Bei weicherer Einstellung weisen die Folien das Phänomen des "kalten Flusses" auf, daß heißt , sie neigen zum gegenseitigen Verkleben. Diese Eigenschaft ist nachteilig, da die Objekte für den Verbraucher dann nicht mehr einzeln anwendbar bzw. dosierbar sind. Der Hauptnachteil ist jedoch darin zu sehen, daß dem Stand der Technik entsprechende aromastoffhaltige Folien, bedingt durch ihren Aufbau und die Hilfsstoffauswahl bei der Herstellung und Lagerung, erhebliche Aroma-Einbußen erleiden. Diese ergeben sich aus dem quantitativen Gesamtverlust von Aromastoffen infolge Migration/Diffusion durch das Grundmaterial und anschließende Verdampfung. Gleichzeitig ändert sich auch die Qualität des Geschmackseindruckes, da leicht flüchtige qualitätsbestimmende Einzelkomponenten bevorzugt verlorengehen.

Ausgehend von diesem Stand der Technik liegt der Erfindung die Aufgabe zugrunde, eine einzeln dosierte Darreichungsform der im gattungsbildenden Oberbegriff von Anspruch 1 angegebenen Art bereitzustellen, welche unter Vermeidung der vorgenannten Nachteile und Schwierigkeiten verbesserte mechanische Eigenschaften und minimalen Aromaverlust bei Herstellung und Lagerung aufweist.

Die Aufgabe wird gemäß der vorliegenden Erfindung entsprechend den Merkmalen von Anspruch 1 gelöst.
Dabei wird die wirkstoffhaltige innere Phase, bei der der Aromastoff in Form von flüssigen Tröpfchen enthalten ist, in einer äußeren, festen, aber wasserlöslichen Phase eingebettet, die entsprechend dem Kennzeichnungsteil von Anspruch 1 Anteile von Polyvinylalkohol, oberflächenaktive Substanzen und Füllstoffe enthält, wobei der Mengenanteil der inneren Phase, bezogen auf die äußere Phase, zwischen 0,1 und 30 Gew.-% liegt. Dabei wird unter Einsatz wesentlicher Anteile von Polyvinylalkohol eine Einbettung des Aromastoffes in der Folie vorgenommen, wobei ein Zweiphasensystem entsteht.

Die erfindungsgemäße Darreichungsform zerfällt im Mund innerhalb von höchstens 5 Minuten, setzt dabei die enthaltenen Aromastoffe frei und macht sie bevorzugt zur Hilfestellung bei kosmetischen, pharmazeutischen und lebensmitteltechnolgischen Anwendungen verfügbar.
Die nach der Erfindung erhaltenen Produkte sind flächenstabil, flexibel und bruchfest sowie weitgehend reißfest. Die adhäsionsmindernden rauhen Oberflächen weisen nur geringe Haftreibung und praktisch keinen "kalten Fluß" auf.

Das erfindungsgemäße Ziel wird regelmäßig dann erreicht, wenn die Außenphase im wesentlichen aus Polyvinylalkohol besteht und der Mengenanteil der aromastoffhaltigen inneren Phase zur äußeren Phase zwischen 0,1 und 30 % (g/g), bevorzugt zwischen 1 und 5 % (g/g), jeweils auf wasserfreie Anteile bezogen, liegt. Unter einem Anteil von 0,1 Gew.-% sind die Phasen ineinander löslich, oberhalb von 30 Gew.-% wird die äußere Phase fettig und ergibt keine Filmbildung mehr.

Zusätze von bis zu 30 % (g/g) einer oberflächenaktiven Substanz zur äußeren Phase können die Gleichmäßigkeit der Verteilung der Tröpfchen und ihre Größe, die zwischen weniger als 1 µm und ca. 100 µm liegen kann, verbessern. Eine Zugabe von bis zu 40 % (g/g) eines Füllstoffes hebt die erfindungsgemäßen Vorteile nicht auf, dehnt aber das Anwendungsgebiet z.B. auch auf einen Einsatz als Trockenzahnpasta aus. Hierzu eigenen sich ohne Anspruch auf erschöpfende Aufzählung Siliciumdioxid, Titandioxid, Calciumcarbonat, Calciumsulfat, Talkum, Calciumphosphat oder Mischungen dieser Stoffe. Aromaunterstützende Stoffe wie Natriumsaccharinat, andere Süßstoffe, Salz und Zuckerderivate sind ebenso zur Verbesserung des geschmacklichen Eindruckes geeignet wie niedermolekulare organische Säuren, z.B. Äpfelsäure, Adipinsäure, Zitronensäure oder Glutaminsäure.
Die hergestellten folienförmigen Produkte weisen bevorzugt eine Dicke zwischen 20 und 300 µm auf, ihre Größe kann vorteilhaft 0,5 bis 8 cm² betragen.

Der verwendete Polyvinylalkohol ist vorteilhafterweise eine teilhydrolysierte Form, bei der zwischen 1 und 20 %, besonders bevorzugt 12 %, der Hydroxylgruppen durch Acetylgruppen ersetzt sind.
Kern der Erfindung ist die Zustandsform des im Grundmaterial enthaltenen Aroma- oder Geruchstoffes und weiterer Geschmacksstoffe. Bei diesen Substanzen handelt es sich im wesentlichen um ätherische Öle (flüchtige, wasserunlösliche Destillate aus duftenden Pflanzenbestandteilen) und andere flüchtige duftende oder Geschmackssubstanzen, die eine begrenzte Mischbarkeit mit Wasser aufweisen. Beispielhaft seien hier z.B. Phenylethanol als Bestandteil von Rosenduftaromen, Menthol, Camphen und Pinen in pfefferminzartig-frischen Aromen, appetitanregende Aromastoffe, Gewürzaromen wie z.B. n-Butylphtalid oder Cineol, aber auch Aromastoffe mit arzneilicher Anwendung wie Eukalyptus- und Thymianöl genannt. Ein sehr weites Feld nehmen Essenzen und/oder Aromen ein, die als Zusätze für Lebensmittel und in vorgefertigten Lebensmittelzusatzstoffen im Einsatz sind. Hier seien beispielhaft die sogenannten Fruchtäther genannt, aber auch andere Aromastoffe wie Äthylvanillin, 6-Methylcumarin, Citronellol oder auch Essigsäure-n-butylester.
Die vorstehend exemplarisch benannten Aromastoffe, die größtenteils untereinander, jedoch nicht mit dem Grundstoff Polyvinylalkohol und auch nicht mit mit Wasser in jedem Verhältnis mischbar sind, liegen erfindungsgemäß im Grundstoff als kleine Tropfen verkapselt eingebettet vor. Dieser Zustand ist dadurch gekennzeichnet, daß sich in einer inneren Phase der Aromastoff in Form winziger Tröpfchen in der festen, ansonsten monolithischen äußeren Phase des getrockneten Polyvinylalkohols und gegebenenfalls weiterer Zuschlagstoffe befindet.
Die tröpfchenförmige Verteilung eines flüssigen Wirkstoffs in einem festen Trägermaterial ist technisch zwar seit langem bekannt, sie findet jedoch bisher ausschließlich in den Verfahren der Koazervation, der Sprühtrocknung, der Sprüherstarrung und Verfahren statt, welche pulverförmige Endprodukte zum Ergebnis haben. Die vorliegende Erfindung beschreibt jedoch einen Verteilungszustand, bei welchem die äußere Phase makroskopisch faßbar und somit ein einfacher, monolithischer Aufbau des Produktes herstellbar ist.

Auch herstellungstechnische Vorteile des erfindungsgemäßen Produktes liegen auf der Hand: Dabei wird vermieden, daß tröpfchenförmige Vorprodukte, die feuchtigkeitsempfindlich sind, bei der Weiterverarbeitung in eine folienartige Darreichungsform in ihrer Integrität gestört werden. Auch werden energieaufwendige Zwischenschritte der Produktion vermieden. Der gleichzeitige Einsatz des Hilfsstoffes Polyvinylalkhol, der durch besonders geringe Diffusibilität für ätherische Öle und andere Aromastoffe gekennzeichnet ist, sichert sowohl bei der Herstellung als auch bei der Lagerung des fertigen Produktes die bestmögliche Konservierung der enthaltenen Aroma- und Geschmacksstoffe sowie deren Sicherung gegen Diffussion aus der Darreichungsform.

Wenn auch die mechanische Festigkeit des System besonders aus der Verwendung von Polyvinylalkohol resultiert, kann ein Anteil von bis zu 20 % anderer wasserlöslicher Polymere für die Qualität des erfindungsgemäßen Produktes unschädlich sein. Vorteilhafte Eigenschaften können im Hinblick auf die Abstimmung der mechanischen Produkteigenschaften auch mit Zugabe von Polyethylenglycol und anderen weichmachenden Zusatzstoffen erreicht werden.

Herstellung und Verarbeitung des erfindungsgemäßen Produktes kann nach dem dem Fachmann bekannten Verfahren durchgeführt werden. Insbesondere wird dazu auf den aus EP 0 460 588 und DE 36 30 603 bekannten Stand der Technik hingewiesen.

In einem bevorzugten Verfahren wird zunächst eine 30%-ige (g/g) Lösung von Polyvinylalkohol in Wasser hergestellt. In diese Phase wird unter langsamem Rühren die vorgewogene Menge von Aroma- bzw. Geschmacksstoff gegeben. Dabei ist hoch scherende Rührbewegung zu vermeiden. Durch Einstellung der Temperatur auf unter 30-40°C und relativ geringe Zusätze von lösungsvermittelnden Zusatzstoffen wird eine Auflösung und Verdunstung der empfindlichen Aroma- bzw. Geschmacksstoffe vermieden. In der Regel ist die flüssige Masse nur einige Stunden lang physikalisch stabil und muß sogleich, bevorzugt in einer Schichtdicke von ca. 200-300 µm, auf einen Träger, z.B. Folienmaterial oder Metallwalze beschichtet und getrocknet werden. Die Trocknung kann in einem Kanaltrockner bei steigenden Temperaturen, die 80°C nicht überschreiten, bis zur gewünschten Produkthärte erfolgen. Wenn eine verringerte Oberflächenhaftung gewünscht ist, kann durch Beschichten auf oberflächenrauhe, dehäsiv beschichtete Materialien eine matte Oberfläche auf dem Produkt erreicht werden.

Sofern nicht Pigmente und andere enthaltene lichtstreuende Zusätze stören, ermöglicht die zweiphasige Struktur überraschenderweise ein transparent bis durchsichtiges Aussehen der Folien. Die Lichtbrechungsindices üblicher Aromastoffe liegen dicht an der Brechkaft von Polyvinylalkohol, so daß keine Lichtstreuwirkung entsteht. Mikroskopisch läßt sich jedoch durch Anfärben der inneren Phase mit lipophilen Farbstoffen, z.B. Sudanrot, jederzeit der disperse Zustand des Aromastoffes nachweisen.

### Beispiel : Herstellung einer erfindungsgemäßen Darreichungsform

17,0 g Polyvinylalkohol (Hydrolysegrad 88 %) werden unter Rühren bei ca. 90°C in 60,0 g Wasser vollständig aufgelöst. Nach dem Erkalten werden 8,0 g Krauseminzöl zugesetzt und 60 min. lang langsam gerührt. Es resultiert eine einheitlich trübe, viskose Masse, die in einer Schichtdicke von 400 µm auf 200 µm starke Polyethylenterephthalat-Folie aufgetragen wird.

Die Schicht trocknet 10 min. bei Raumtemperatur vor und wird anschließend bei 50°C 8,0 min. lang nachgetrocknet. Es resultiert eine klar durchsichtige, monolithisch erscheinende Folie, welche sich bei Wasserzutritt innerhalb von 60 Sekunden vollständig auflöst. Nach Äquilibrierung mit 60% relativer Feuchte über 24 Stunden bleibt die Folie beständig gegen einen Biegeradius von 1 mm. Die Oberfläche ist trocken und gleitfähig und ermöglicht dauerhaft die Aufbewahrung in Stapelform.
Nach 1 Woche unverpackter Lagerung bei 25°C/60% rel. Feuchte bleibt der Geschmackseindruck subjektiv einwandfrei.

Die Erfindung wird nachfolgend in einer Figur näher erläutert:

Es bedeuten, im Querschnitt einer erfindungsgemäßen Darreichungsform gesehen:
1 - Äußere, wasserlösliche Phase der folienförmigen Darreichungsform.
2 - Innere, fettlösliche Phase, enthaltend Aroma- oder Geschmacksstoffe.

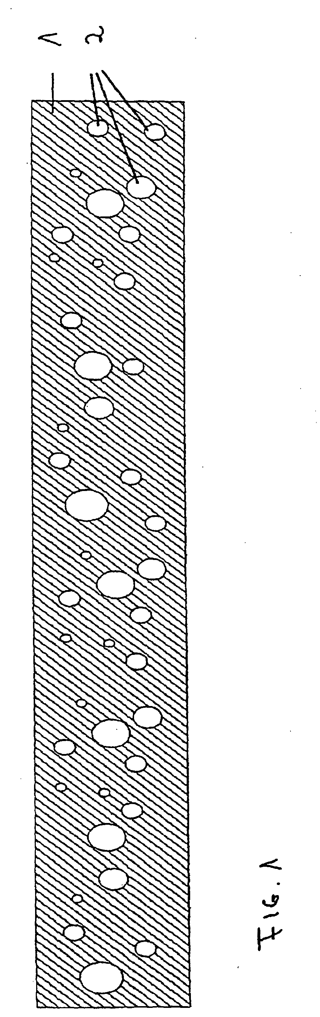

## Patentansprüche

1. Einzeln dosierte, bei Kontakt mit Flüssigkeit schnell zerfallende, wirkstoff- und insbesondere aromastoffhaltige, folienförmige Darreichungsform, bei der sich der Aromastoff als innere, fettlösliche Phase in Form von flüssigen Tröpfchen verteilt in einer äußeren, festen aber wasserlöslichen Phase befindet, **dadurch gekennzeichnet, daß** die äußere Phase enthält:
- mindestens 40% (g/g) Polyvinylalkohol
- 0 bis 30 % (g/g) einer oberflächenaktiven Substanz,und daß der Mengenanteil der inneren Phase, bezogen auf die äußere Phase, zwischen 0,1 und 30% (g/g), jeweils auf wasserfreie Anteile bezogen, liegt.

2. Darreichungsform nach Anspruch 1, **dadurch gekennzeichnet, daß** die äußere Phase bis zu 40% (g/g) eines Füllstoffs enthält.

3. Darreichungsform nach Anspruch 1 oder 2, **dadurch gekennzeichnet, daß** der Mengenanteil der inneren Phase, bezogen auf die äußere Phase, zwischen 1 und 5% (g/g) liegt, jeweils auf wasserfreie Anteile bezogen.

4. Darreichungsform nach Anspruch 3, **dadurch gekennzeichnet, daß** der Füllstoff aus Siliciumdioxid, Titandioxid, Calciumcarbonat, Calciumsulfat, Talkum, Calciumphosphat oder aus Mischungen dieser Stoffe besteht.

5. Darreichungsform nach einem oder mehreren der Ansprüche 1 bis 4, **dadurch gekennzeichnet, daß** sie als Folie mit einer Dicke zwischen 20 und 300 Micrometer vorliegt.

6. Darreichungsform nach einem oder mehreren der Ansprüche 1 bis 5, **dadurch gekennzeichnet, daß** sie als Folie mit einer Fläche zwischen 0,5 und 8 cm² vorliegt.

7. Darreichungsform nach einem oder mehreren der Ansprüche 1 bis 6, **dadurch gekennzeichnet, daß** sie mindestens einseitig eine adhäsionsmindernde rauhe Oberfläche aufweist.

8. Darreichungsform nach Anspruch 1, **dadurch gekennzeichnet, daß** der Polyvinylalkohol in einer teilhydrolysierter Form vorliegt, in der bis zu 20% der Hydroxylgruppen durch Acetylgruppen ersetzt sind.

9. Darreichungsform nach Anspruch 1, **dadurch gekennzeichnet, daß** sie bis zu 20 % wasserlösliche Polymere enthält.

10. Darreichungsform nach Anspruch 1, **dadurch gekennzeichnet, daß** sie weichmachende Zusatzstoffe enthält.

## Claims

1. Individually dosed active substance-containing and, in particular, aromatic-containing, film-shaped administration form, rapidly disintegrating upon contact with a liquid, wherein the aromatic is present as an internal, liposoluble phase in the form of liquid droplets distributed within an outer, solid but water-soluble phase, **characterized in that** the said outer phase contains:
- at least 40% (w/w) polyvinyl alcohol
- 0 to 30% (w/w) of a surface-active substance, and that the constituent amount of the inner phase, relative to the outer phase, is between 0.1 and 30% (w/w), in each case relative to the water-free portions.

2. Administration form according to claim 1, **characterized in that** the outer phase contains up to 40% (w/w) of a filling agent.

3. Administration form according to claim 1 or 2, **characterized in that** the constituent amount of the inner phase, relative to the outer phase, is between 1 and 5% (w/w), in each case relative to water-free portions.

4. Administration form according to claim 3, **characterized in that** the filling agent consists of silicon dioxide, titanium dioxide, calcium carbonate, calcium sulfate, talcum, calcium phosphate or of mixtures of these substances.

5. Administration form according to one or more of claims 1 to 4, **characterized in that** it is present as a film with a thickness of between 20 and 300 micrometers.

6. Administration form according to one or more of claims 1 to 5, **characterized in that** it is present as a film having an area between 0.5 and 8 cm².

7. Administration form according to one or more of claims 1 to 6, **characterized in that** it has an adhesion-reducing, rough surface on at least one side thereof.

8. Administration form according to claim 1, **characterized in that** the said polyvinyl alcohol is present in a partially hydrolized form, wherein up to 20% of the hydroxyl groups are replaced by acetyl groups.

9. Administration form according to claim 1, **characterized in that** it contains up to 20% water-soluble polymers.

10. Administration form according to claim 1, **characterized in that** it contains plasticizing additives.

## Revendications

1. Forme galénique en forme de feuilles à dosage individuel, se désagrégeant rapidement au contact avec un liquide, contenant des substances actives et en particulier des substances aromatiques, dans laquelle la substance aromatique se présente en tant que phase intérieure soluble dans les graisses, sous la forme de gouttelettes liquides réparties dans une phase extérieure solide mais soluble dans l'eau, **caractérisée en ce que** la phase extérieure contient au moins 40 % (poids/poids) d'alcool polyvinylique, de 0 à 30 % (poids/poids) d'une substance tensioactive, et **en ce que** la proportion de la phase intérieure par rapport à la phase extérieure est comprise entre 0,1 et 30 % (poids/poids), chaque fois par rapport aux quantités exemptes d'eau.

2. Forme galénique selon la revendication 1, **caractérisée en ce que** la phase extérieure contient jusqu'à 40 % (poids/poids) d'une charge.

3. Forme galénique selon la revendication 1 ou 2, **caractérisée en ce que** la proportion de la phase intérieure par rapport à la phase extérieure est comprise entre 1 et 5 % (poids/poids), chaque fois par rapport à la composition exempte d'eau.

4. Forme galénique selon la revendication 3, **caractérisée en ce que** la charge est constituée de dioxyde de silicium, de dioxyde de titane, de carbonate de calcium, de sulfate de calcium, de talc, de phosphate de calcium ou de mélanges de ces substances.

5. Forme galénique selon l'une ou plusieurs des revendications 1 à 4, **caractérisée en ce qu'**elle se présente sous la forme d'une feuille d'une épaisseur comprise entre 20 et 300 micromètres.

6. Forme galénique selon l'une ou plusieurs des revendications 1 à 5, **caractérisée en ce qu'**elle se présente sous la forme d'une feuille d'une surface comprise entre 0,5 et 8 cm².

7. Forme galénique selon l'une ou plusieurs des revendications 1 à 6, **caractérisée en ce qu'**au moins d'un côté, elle présente une surface rugueuse qui diminue l'adhérence.

8. Forme galénique selon la revendication 1, **caractérisée en ce que** l'alcool polyvinylique se présente sous une forme partiellement hydrolysée dans laquelle jusqu'à 20 % des groupes hydroxyle sont substitués par des groupes acétyle.

9. Forme galénique selon la revendication 1, **caractérisée en ce que** qu'elle contient jusqu'à 20 % de polymères solubles dans l'eau.

10. Forme galénique selon la revendication 1, **caractérisée en ce qu'**elle contient des additifs plastifiants.
